# EUROPÄISCHE PATENTANMELDUNG

(11) **EP 4 345 086 A1**
(43) Veröffentlichungstag der Anmeldung: **03.04.2024**
(21) Anmeldenummer: 22020473.9
(22) Anmeldetag: 30.09.2022
(51) Int. Cl.: C07C 29/151, C07C 31/04, C25B 1/04

(54) **VERFAHREN UND ANLAGE ZUR HERSTELLUNG VON METHANOL**

(71) Anmelder: Linde GmbH, 82049 Pullach (DE); Technische Universität München, 80333 München (DE)
(72) Erfinder: Peschel, Andreas, 82049 Pullach (DE); Klein, Harald, 80290 München (DE); Hemauer, Johanna, 80290 München (DE); Meier, Carolin, 82049 Pullach (DE)
(74) Vertreter: Fischer, Werner

(57) **Zusammenfassung**

Die Erfindung betrifft ein Verfahren (1000, 2000) zur Herstellung von Methanol, bei dem ein Wasserstoff und Kohlendioxid enthaltender Methanolsyntheseeinsatz (101) unter Bildung eines Methanol, tiefer als Methanol siedende Komponenten und höher als Methanol siedende Komponenten enthaltenden Rohproduktgemischs (103) einer Methanolsynthese (100) unterworfen wird, wobei zumindest ein Teil des Rohproduktgemischs (103) unter Bildung eines Methanolprodukts (405) und einer oder mehrerer, gegenüber dem Rohproduktgemisch (103) an Methanol abgereicherter und an zumindest einer der tiefer als Methanol siedenden Komponenten angereicherter Leichtgasfraktionen (401, 403) einer Methanolaufreinigung (400) unterworfen wird, wobei zumindest ein Teil des Wasserstoffs in dem Methanolsyntheseeinsatz (101) unter Verwendung einer Elektrolyse (200, 500) bereitgestellt wird, und wobei zumindest ein Teil der Leichtgasfraktion (401, 403) oder zumindest ein Teil zumindest einer der mehreren Leichtgasfraktionen (401, 403) in die Elektrolyse (200, 500) oder einen weiteren, zur Bereitstellung des Methanolsyntheseeinsatzes (101) verwendeten Verfahrensschritt (310) zurückgeführt wird. Eine entsprechende Anlage ist ebenfalls Gegenstand der vorliegenden Erfindung.

## Beschreibung

Die Erfindung betrifft ein Verfahren und eine Anlage zur Herstellung von Methanol.

### Hintergrund

Die Herstellung von Methanol in großem Maßstab erfolgt herkömmlicherweise aus Synthesegas gemäß folgenden Gleichungen:

| | | |
|---|---|---|
| 2 H₂ + CO 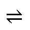 MeOH | ΔH = -98 kJ/mol | (1) |
| 3 H₂ + CO₂ 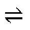 MeOH + H₂O | ΔH = -58 kJ/mol | (2) |

Synthesegas, worunter nachfolgend ein Gasgemisch verstanden werden soll, das Wasserstoff, Kohlenmonoxid und optional Kohlendioxid aufweist und insbesondere überwiegend, d.h. zu mehr als 50, 60, 70, 80, 90, 95, 99 oder 99,9% im Volumen- oder Mengenanteil aus diesen Komponenten besteht, wird herkömmlicherweise aus fossilen Quellen wie Erdgas oder vergaster Kohle gewonnen. Auch eine Herstellung ausgehend von nichtfossilen Quellen wie Biogas oder Abfällen ist möglich.

Die Herstellung von Synthesegas kann beispielsweise mittels Dampfreformierung, autothermer Reformierung oder durch partielle Oxidation erfolgen. Auf einschlägige Fachliteratur wie Baerns, M. et al.: Technische Chemie, 2. Aufl., Wiley VCH, Weinheim 2013, oder Rostrup-Nielsen, J. & Christiansen, L.J.: Concepts in Syngas manufacture, 2011, Imperial College Press, London 2011, wird verwiesen.

Ein Gesamtverfahren zur Herstellung von Methanol, auch als "Methanolprozess" bezeichnet, besteht aus zwei Hauptschritten, der eigentlichen Methanolsynthese, bei der Rohmethanol erzeugt wird, und einer Reinigung, die mittels Rektifikation durchgeführt wird. Die Reinigung umfasst i.d.R. die Verwendung einer sogenannten Toppingkolonne, die dazu dient, leichte Gase und nicht umgewandelte Edukte über Kopf zu entfernen. Die verbleibende flüssige Sumpffraktion wird einer sogenannten Raffinationskolonne zugeführt, in der über Kopf Methanol gewonnen wird.

Sowohl im konventionellen als auch im grünen Methanolprozess (siehe sogleich) existieren zwei Quellen für Abgase: Nach der Entnahme aus dem Synthesereaktor wird der Prozessstrom gekühlt und geflasht. Das dabei gebildete Flashgas, das nicht umgewandelte Edukte enthält, wird in den Reaktor zurückgeführt. Ein Teil davon muss jedoch ausgeleitet (gepurget) werden, um Inertstoffe und Nebenprodukte zu entfernen und eine Anreicherung im Kreislauf zu vermeiden. Daneben wird ein Abgasstrom in Form des leichten Endes der Toppingkolonne (und/oder einem Niederdruck-Flashbehälter stromauf der Toppingkolonne) gebildet. In Kombination mit z.B. einem Dampfreformer zur Erzeugung von Synthesegas können beide Abgasströme verfeuert werden und enden in solchen Fällen als Rauchgas.

Die direkte Hydrierung von Kohlendioxid zu grünem Methanol über die oben angegebene Reaktion (2) auf der Grundlage der Wasserelektrolyse zur Wasserstoffproduktion unter Verwendung erneuerbarer Energien stößt im letzten Jahrzehnt im Zusammenhang mit sogenannten Power-to-X-Anwendungen auf zunehmendes Interesse. Durch die vielversprechende Entwicklung von Katalysatoren ist dies ein nachhaltiger Ansatz zur Verringerung von Kohlendioxidemissionen.

Die Kopplung des Methanolprozesses mit einer Elektrolyse schafft neue Möglichkeiten für die Systemintegration, insbesondere im Hinblick auf die Nutzung der Abgasströme.

### Offenbarung der Erfindung

Vor diesem Hintergrund werden ein Verfahren und eine Anlage zur Herstellung von Methanol mit den jeweiligen Merkmalen der unabhängigen Patentansprüche vorgeschlagen. Vorteilhafte Ausgestaltungen der vorliegenden Erfindung sind Gegenstand der abhängigen Patentansprüche und der nachfolgenden Beschreibung.

Ein Verfahren zur Herstellung von Methanol gemäß einer Ausgestaltung der Erfindung umfasst, dass ein Wasserstoff und Kohlendioxid enthaltender Methanolsyntheseeinsatz unter Bildung eines Methanol, tiefer als Methanol siedende Komponenten und höher als Methanol siedende Komponenten enthaltenden Rohproduktgemischs einer Methanolsynthese unterworfen wird, wobei zumindest ein Teil des Rohproduktgemischs unter Bildung eines Methanolprodukts und einer oder mehrerer, gegenüber dem Rohproduktgemisch an Methanol abgereicherter und an zumindest einer der tiefer als Methanol siedenden Komponenten angereicherter Leichtgasfraktionen einer Methanolaufreinigung unterworfen wird, wobei zumindest ein Teil des Wasserstoffs in dem Methanolsyntheseeinsatz unter Verwendung einer Elektrolyse bereitgestellt wird, und wobei zumindest ein Teil der Leichtgasfraktion oder zumindest ein Teil zumindest einer der mehreren Leichtgasfraktionen in die Elektrolyse oder einen weiteren, zur Bereitstellung des Methanolsyntheseeinsatzes verwendeten Verfahrensschritt zurückgeführt wird.

Ausgestaltungen der vorliegenden Erfindung umfassen damit insbesondere, Abgase, d.h. die genannte(n) Leichtgasfraktion(en), aus dem Methanolprozess rückzuführen, wodurch insbesondere die Kohlendioxidemissionen verringert und die Kohlenstoffeffizienz im Vergleich zu herkömmlichen Methanolprozessen erhöht werden können. Aufgrund der geringeren Nebenproduktrate bei der Direkthydrierung von Kohlendioxid kann in Ausgestaltungen der vorliegenden Erfindung eine Einkolonnentrennung erfolgen, die den Reinigungsschritt vereinfacht und gleichzeitig die Qualität des Methanols gewährleistet. Insbesondere kann auf diese Weise Methanol der Reinheit "AA" erzeugt werden. Eine Rektifikationseinheit (Rektifikationskolonne) kann in diesem Fall nahe ihres oberen Endes einen Methanol-Seitenabzug und einem Fuselölseitenabzug weiter unten aufweisen. Letzter befindet sich insbesondere dort, wo eine Ethanolanreicherung erkennbar ist bzw. erfolgt. Leichte Endgase werden am Kopf der Kolonne entfernt und eine wasserreiche Fraktion verbleibt am Boden.

Die vorliegende Erfindung kann dabei insbesondere mit unterschiedlichen Arten einer Hochtemperaturelektrolyse, aber auch mit anderen Elektrolyseverfahren, beispielsweise unter Einsatz von Protonenaustauschmembranen, zum Einsatz kommen. Solche Verfahren sind in der Fachliteratur, beispielsweise im Artikel "Hydrogen" in Ullmann's Encyclopedia of Industrial Chemistry, 15. Juni 2000, DOI: 10.1002/14356007.a13_297, Abschnitt 4.2, "Electrolysis", erläutert. Bei der Herstellung von Wasserstoff durch Elektrolyse handelt es sich derzeit um das vielversprechendste Verfahren der Wasserstofferzeugung aus Wasser.

Bei der alkalischen Wasserelektrolyse wird eine wässrige alkalische Lösung, typischerweise von Kaliumhydroxid, als Elektrolyt verwendet (AEL, alkalische Elektrolyse). Die Elektrolyse mit einer uni- oder bipolaren Elektrodenanordnung erfolgt hierbei bei Atmosphärendruck oder im industriellen Maßstab auch bei einem Überdruck von bis zu 30 bar. Neuere Entwicklungen bei der Wasserelektrolyse umfassen die Verwendung von anionen- oder protonenleitenden lonenaustauschmembranen bzw. Anionen- oder Protonenaustauschmembranen (AEM, Anion Exchange Membrane; PEM, Proton Exchange Membrane). Die genannten Verfahren zählen zu den sogenannten Niedertemperaturverfahren, bei denen das zu elektrolysierende Wasser in der Flüssigphase vorliegt.

Die Herstellung von Wasserstoff unter Verwendung von Hochtemperatur- bzw. Festoxidelektrolysezellen (engl. Solid Oxide Electrolysis Cells, SOEC) ist ebenfalls bekannt. In derartigen Elektrolysezellen wird Wasserdampf (und/oder Kohlendioxid) unter Verwendung eines Festoxid- oder Keramikelektrolyten zu Wasserstoff (und/oder Kohlenmonoxid) und Sauerstoff umgesetzt werden. Vorteil der Hochtemperaturelektrolyse ist ein niedriger elektrischer Energieverbrauch verglichen mit den Niedertemperaturverfahren.

Der technische Hintergrund und Ausgestaltungen entsprechender Elektrolysen ist beispielsweise in den Absätzen [0019] bis [0024] der EP 3 766 831 A1 beschrieben, auf die an dieser Stelle ausdrücklich Bezug genommen wird, und die hier durch Bezugnahme in vollem Umfang aufgenommen werden.

Zusammengefasst reagiert in der Hochtemperaturelektrolyse mit einer sauerstoffionenleitenden Festoxidelektrolysezelle an der Kathode ein Wassermolekül mit zwei Elektronen zu einem Wasserstoffmolekül und einem Sauerstoffion. An der Anode reagiert ein Sauerstoffion zu (mathematisch) einem halben Sauerstoffmolekül und zwei Elektronen. Die Sauerstoffionen sind die Ladungsträger.

Zur elektrochemischen Herstellung von Kohlenmonoxid aus Kohlendioxid kann ebenfalls eine Hochtemperaturelektrolyse, die unter Verwendung einer oder mehrerer Festoxid-Elektrolysezellen durchgeführt wird, zum Einsatz kommen. Hierbei bilden sich Sauerstoff auf der Anodenseite und Kohlenmonoxid auf der Kathodenseite. Beispielsweise wird auf die WO 2014/154253 A1, die WO 2013/131778 A2, die WO 2015/014527 A1 und die EP 2 940 773 A1 verwiesen.

Auch eine Koelektrolyse von Wasser und Kohlendioxid in entsprechenden Einrichtungen ist möglich. An der Kathode werden in derartigen Verfahren Wasserstoff, und Kohlenmonoxid gebildet. Auch eine Koelektrolyse kann im Rahmen der vorliegenden Erfindung eingesetzt werden. Man erhält dann ein Wasserstoff, Kohlenmonoxid und Kohlendioxid enthaltendes Produkt (d.h. sogenanntes Synthesegas).

Gemäß einer Ausgestaltung der vorliegenden Erfindung kann die Elektrolyse also als Hochtemperaturelektrolyse durchgeführt werden, der Dampf zugeführt wird. Insbesondere werden in der Hochtemperaturelektrolyse dabei keine weiteren Komponenten als Wasser bzw. Wasserdampf elektrolysiert, so dass es sich um eine reine Wasserelektrolyse bzw. Dampfelektrolyse handelt.

In entsprechenden Ausgestaltungen der vorliegenden Erfindung umfasst der Dampf einen Dampfanteil, der unter Verwendung von Dampf gebildet wird, der bei einer Kühlung einer in der Methanolsynthese eingesetzten Methanolsyntheseeinheit aus Kühlwasser erzeugt wird. Auf diese Weise ergibt sich eine besonders vorteilhafte Wärmeintegration, wobei überschüssiger Dampf auch auf andere Weise genutzt werden kann, beispielsweise für eine Beheizung eines Sumpfverdampfers einer Rektifikationseinheit oder dergleichen.

Ferner kann in entsprechenden Ausgestaltungen der vorliegenden Erfindung die Elektrolyse als Hochtemperaturelektrolyse von Wasser durchgeführt werden, wobei der Methanolsyntheseeinsatz unter Verwendung von Wasserstoff, der in der Elektrolyse erzeugt wird, und verfahrensextern bereitgestelltes Kohlendioxid, das nicht der Elektrolyse unterworfen wurde, umfassen kann. Somit erlaubt eine derartige Ausgestaltung der Erfindung eine unabhängige Bereitstellung der Komponenten Wasserstoff und Kohlendioxid und ggf. auch eine separate Zwischenspeicherung zumindest einer dieser Komponenten. Auf diese Weise können die Bereitstellung der Edukte und die Durchführung der Methanolsynthese vorteilhaft entkoppelt werden.

In derartigen Ausgestaltungen kann das verfahrensextern bereitgestellte Kohlendioxid einer Kohlendioxidverdichtung unterworfen werden, wobei zumindest ein Teil der Leichtgasfraktion oder zumindest ein Teil zumindest einer der mehreren Leichtgasfraktionen in die Kohlendioxidverdichtung zurückgeführt wird. Auf diese Weise können auch weitere Komponenten aus einer entsprechenden Leichtgasfraktion außer Kohlendioxid genutzt werden. Auch eine Aufbereitung einer entsprechenden Leichtgasfraktion kann vorgesehen sein.

In alternativen Ausgestaltungen zur reinen Wasserelektrolyse kann auch vorgesehen sein, dass eine Hochtemperaturkoelektrolyse von Wasser und Kohlendioxid durchgeführt wird, wobei unter Verwendung der Elektrolyse ein Wasserstoff, Kohlenmonoxid und Kohlendioxid enthaltendes Synthesegas bereitgestellt und zur Bereitstellung des Methanolsyntheseeinsatzes verwendet wird. Auf diese Weise kann insbesondere auch ein kombiniertes Komponentengemisch als Methanolsyntheseeinsatz bearbeitet werden. Ein Vorteil einer derartigen Ausgestaltung liegt in der Möglichkeit der Verwendung des konventionellen und etablierten Katalysators, wie er in Prozessen mit Kopplung, z.B. einer Dampfreformierung, eingesetzt wird. Auf diese Weise können (mögliche) Stabilitätsprobleme gegenüber dem gebildeten Wasser gemäß der oben angegebenen Reaktion (2), wodurch der Katalysator schneller deaktiviert werden kann, sicher vermieden werden.

Derartige Ausgestaltungen können insbesondere umfassen, dass zumindest ein Teil der Leichtgasfraktion oder zumindest ein Teil zumindest einer der mehreren Leichtgasfraktionen der Hochtemperaturkoelektrolyse von Wasser und Kohlendioxid kathodenseitig zugeführt wird. Dies erlaubt insbesondere die Umsetzung von darin enthaltenem Kohlendioxid zu Kohlenmonoxid bzw. die Beeinflussung des Wasserstoffgehalts in dem Einsatzgemisch.

Dabei kann insbesondere vorgesehen sein, dass zumindest ein Teil der Leichtgasfraktion oder zumindest ein Teil zumindest einer der mehreren Leichtgasfraktionen vor der kathodenseitigen Zuführung zu der Hochtemperaturkoelektrolyse einem Präreformierungsschritt unterworfen wird. Auf diese Weise können insbesondere Komponenten umgesetzt werden, die ggf. die Elektrolysezelle(n) schädigen könnten, bspw. Kohlenwasserstoffe.

Erdgas oder ein anderes kohlenwasserstoffhaltiges Gasgemisch kann in entsprechenden Ausgestaltungen ebenfalls dem Verfahren zugeführt werden und dabei, kathoden- und/oder anodenseitig in die Hochtemperaturkoelektrolyse eingespeist werden. Eine kathodenseitige Zuführung erlaubt dabei die Gewinnung von Elektrolyseprodukten, eine anodenseitige Zuführung eine thermische Nutzung, d.h. die Bereitstellung von Wärme für die Hochtemperaturelektrolyse u.a. aus anfallenden Abgasströmen im Methanolprozess. Bei Bedarf, und insbesondere dann, wenn eine Elektrolysezelle sensibel auf Kohlenwasserstoffe reagiert, kann eine Präreformierung zum Einsatz können. Mit anderen Worten können also Abgasströme bzw. Teile hiervon als zumindest ein Teil der Leichtgasfraktion oder zumindest ein Teil zumindest einer der mehreren Leichtgasfraktionen verwendet werden.

In Ausgestaltungen der vorliegenden Erfindung kann die Methanolaufreinigung unter Verwendung einer einzigen oder mehrerer, parallel betriebener Rektifikationseinheiten durchgeführt werden, der oder denen das Methanolprodukt über einen Seitenabzug entnommen wird. Wie bereits erläutert, erlaubt eine derartige Lösung eine besonders einfache und kostengünstige Realisierung der Trennung.

Der einen oder den mehreren Rektifikationseinheiten kann eine Flasheinheit vorgeschaltet sein, in der ein bei einer Abkühlung des Rohproduktgemischs gebildetes Kondensat unter Erhalt eines Flashgases und einer Flüssigfraktion geflasht wird, wobei zumindest ein Teil der Flüssigfraktion der einen oder den mehreren Rektifikationseinheiten zugeführt wird.

Ein Kopfgas der einen oder der mehreren Rektifikationseinheiten kann in entsprechenden Ausgestaltungen unter Verbleib einer Gasphase teilkondensiert werden, wobei zumindest ein Teil der Gasphase die oder eine der zuvor erläuterten Leichtgasfraktionen ist. Ausgestaltungen der vorliegenden Erfindung erlauben dabei eine besonders vorteilhafte Nutzung bzw. Rückführung.

Der einen oder den mehreren Rektifikationseinheiten kann unterhalb des Methanolprodukts über einen weiteren Seitenabzug ein Fuselöle enthaltender Strom und sumpfseitig Abwasser entnommen werden.

Eine Anlage zur Herstellung von Methanol ist ebenfalls Gegenstand der vorliegenden Erfindung, wobei die Anlage die dafür eingerichtet ist, einen Wasserstoff und Kohlendioxid enthaltenden Methanolsyntheseeinsatz unter Bildung eines Methanol, tiefer als Methanol siedende Komponenten und höher als Methanol siedende Komponenten enthaltenden Rohproduktgemischs einer Methanolsynthese zu unterwerfen, zumindest einen Teil des Rohproduktgemischs unter Bildung eines Methanolprodukts und einer oder mehrerer, gegenüber dem Rohproduktgemisch an Methanol abgereicherter und an zumindest einer der tiefer als Methanol siedenden Komponenten angereicherter Leichtgasfraktionen einer Methanolaufreinigung zu unterwerfen, zumindest einen Teil des Wasserstoffs in dem Methanolsyntheseeinsatz unter Verwendung einer Elektrolyse bereitzustellen, und zumindest einen Teil der Leichtgasfraktion oder zumindest ein Teil zumindest einer der mehreren Leichtgasfraktionen in die Elektrolyse oder einen weiteren, zur Bereitstellung des Methanolsyntheseeinsatzes verwendeten Verfahrensschritt zurückzuführen.

Zu weiteren Merkmalen und Vorteilen einer entsprechenden Anlage und Ausgestaltungen hiervon sei auf die obigen Erläuterungen betreffend das erfindungsgemäß vorgeschlagene Verfahren und seine Ausgestaltungen ausdrücklich verwiesen, da diese hierfür in gleicher Weise gelten.

Entsprechendes gilt auch für eine Anlage, die gemäß einer Ausgestaltung der Erfindung dazu eingerichtet ist, ein Verfahren gemäß einer beliebigen Ausgestaltung der vorliegenden Erfindung durchzuführen.

Nachfolgend werden Ausgestaltungen der vorliegenden Erfindung und weitere Ausgestaltungen bzw. Aspekte hiervon, teilweise in Wiederholung des zuvor Gesagten, und teilweise unter Nennung neuer Merkmale und Vorteile, erläutert.

Ein Spül- bzw. Purgestrom aus dem Methanolkreislauf und das Abgas aus der Raffinationssäule, d.h. einer Leichtgasfraktion einer Zweikolonneneinheit kann auf verschiedene Weise verwendet oder verarbeitet werden. Beispielsweise kann ein Export und z.B. eine Verwendung als Brennstoff in anderen Prozessen erfolgen. Auch kann das Abgas in einer Hochtemperatur- oder Feststoffoxidbrennstoffzelle (engl. Solid Oxide Fuel Cell, SOFC) anodenseitig zur Stromerzeugung oder auf der Anodenseite der Hochtemperaturelektrolyse (engl. Solid Oxide Fuel-Assisted Electrolyzer Cell, SOFEC) thermisch verwertet werden. Wasserstoff (aus entsprechenden Spülströmen) kann mit einer Druckwechseladsorption zurückgewonnen und entweder in den Methanolreaktor zurückgeführt oder in die Elektrolyse geleitet werden, um dort reduzierende Bedingungen aufrechtzuerhalten, so dass kein Wasserstoff vom Ausgang der Elektrolyse zurückgeführt werden muss.

Wasserstoff kann auch mit einer Membrantrennung zurückgewonnen und das Niederdruckpermeat (hauptsächlich Wasserstoff) in die Elektrolyse geleitet werden, um dort reduzierende Bedingungen aufrechtzuerhalten.

Aufgrund von Degradations-/Verkokungsproblemen kann ein Präreformer vor der anodenseitigen Zuführung zur Dampfelektrolyse vorteilhaft sein, um schwerere Komponenten zu reformieren. Bei einer Festoxidelektrolysezelle kann ein Präreformer anodenseitig, bei einer Festoxidbrennstoffzelle anodenseitig, und bei einer Koelektrolyse unter Verwendung einer Festoxidelektrolysezelle anoden- und kathodenseitig vorgesehen sein und eine entsprechende Zuführung erfolgen. In solchen Fällen kann das Fuselöl ebenfalls in den Präreformer zurückgeführt werden. In der Festoxidbrennstoffzelle kann so Elektrizität aus Abgasen und/oder Fuselöl erzeugt werden. Eine Wiederverwendung auf der Kathodenseite einer Koelektrolyse ist möglich (mit Präreformer), um Strom zu sparen und die Kohlenstoffeffizienz zu erhöhen.

Aus einem Spülstrom wie beispielsweise einem Methanolrecycle müssen immer Inertstoffe wie Stickstoff und Argon entfernt werden, um eine Anreicherung im Kreislauf zu vermeiden. Hierfür können beliebige Trennschritte vorgesehen sein.

Abgas einer Raffinationskolonne in einer Zweikolonnenanordnung kann nach Kondensation des Methanolprodukts in den Methanolkreislauf, in die Toppingkolonne, anodenseitig zur Dampfelektrolyse und zur Festoxidbrennstoffzelle oder kathoden- und/oder anodenseitig zur Festoxid-Koelektrolysezelle recycelt werden.

Die Integration verschiedener Elektrolysetechnologien mit einer nachgeschalteten Methanolanlage, wie sie gemäß Ausgestaltungen der vorliegenden Erfindung vorgesehen ist, hat eine Reihe von Vorteilen. Die gasförmige Fraktion aus dem oberen Teil der Reinigungssäule (eine Leichtgasfraktion) wird recycelt, anstatt wie beim herkömmlichen Verfahren verbrannt zu werden, so dass die Kohlenstoffeffizienz steigt und die Kohlendioxidemissionen gemindert werden. Der Einsatz von einer statt zwei Rektifikationseinheiten führt zu einer Senkung der Investitionskosten. Es verbleiben nur der Reaktorreinigungsrücklauf, das Abgas aus der Raffinationssäule (falls zwei Säulen erforderlich sind) und ein Fuselölabzug, falls dieser nicht im Prozess verwendet wird, was verschiedene (nachhaltigere) Anwendungsfälle als die Verbrennung ermöglicht. Zur Erzeugung von Dampf für einen Reboiler einer Rektifikationseinheit kann beispielsweise eine Wärmepumpe installiert werden, die die Kühlleistung des Kondensators der Kolonne, die Kühlleistung der Anoden- und Kathodenauslassströme (in einer Hochtemperaturelektrolyse), die Kühlleistung der in einer Elektrolyse mit Protonenaustauschmembran und/oder die Kühlleistung des/der Kondensators/Kondensatoren im Methanolkreislauf nutzt.

### Kurze Beschreibung der Zeichnung

Ausführungsformen der Erfindung werden nachfolgend rein beispielhaft unter Bezugnahme auf die beigefügte Zeichnung beschrieben, wobei
Figuren 1 und 2 Verfahren zur Herstellung von Methanol gemäß Ausgestaltungen der vorliegenden Erfindung veranschaulichen, und
Figur 3 eine Rektifikation zum Einsatz gemäß einer Ausgestaltung der vorliegenden Erfindung veranschaulicht.

### Ausführungsformen der Erfindung

Die nachfolgend beschriebenen Ausführungsformen werden lediglich zu dem Zweck beschrieben, den Leser beim Verständnis der beanspruchten und zuvor erläuterten Merkmale zu unterstützen. Sie stellen lediglich repräsentative Beispiele dar und sollen hinsichtlich der Merkmale der Erfindung nicht abschließend und/oder beschränkend betrachtet werden. Es versteht sich, dass die zuvor und nachfolgend beschriebenen Vorteile, Ausführungsformen, Beispiele, Funktionen, Merkmale, Strukturen und/oder andere Aspekte nicht als Beschränkungen des Umfangs der Erfindung, wie er in den Ansprüchen definiert ist, oder als Beschränkungen von Äquivalenten zu den Ansprüchen zu betrachten sind, und dass andere Ausführungsformen verwendet und Änderungen vorgenommen werden können, ohne vom Umfang der beanspruchten Erfindung abzuweichen.

Unterschiedliche Ausführungsformen der Erfindung können weitere zweckmäßige Kombinationen der beschriebenen Elemente, Komponenten, Merkmale, Teile, Schritte, Mittel usw. umfassen, aufweisen, aus ihnen bestehen oder im Wesentlichen aus ihnen bestehen, auch wenn solche Kombinationen hier nicht spezifisch beschrieben sind. Darüber hinaus kann die Offenbarung andere Erfindungen umfassen, die gegenwärtig nicht beansprucht sind, die aber in Zukunft beansprucht werden können, insbesondere wenn sie vom Umfang der unabhängigen Ansprüche umfasst sind.

Erläuterungen, die sich auf Vorrichtungen, Apparate, Anordnungen, Systeme usw. gemäß Ausführungsformen der vorliegenden Erfindung beziehen, können auch für Verfahren, Prozesse, Methoden usw. gemäß den Ausführungsformen der vorliegenden Erfindung gelten und umgekehrt. Gleiche, gleich wirkende, in ihrer Funktion einander entsprechende, baulich identisch oder vergleichbar aufgebaute Elemente, Verfahrensschritte usw. können mit identischen Bezugszeichen angegeben sein.

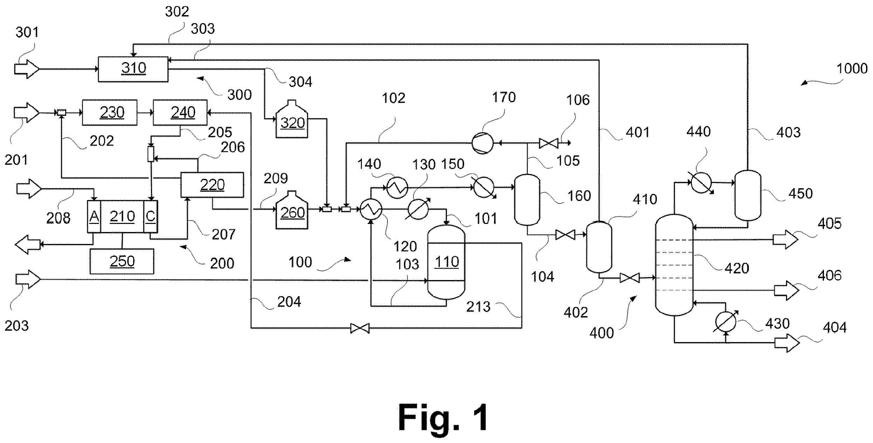

In Figur 1 ist ein Verfahren 1000 gemäß einer Ausgestaltung der Erfindung veranschaulicht, das eine Hochtemperaturelektrolyse von Wasser umfasst.

Verfahren 1000 umfasst eine insgesamt mit 100 bezeichnete Methanolsynthese, eine insgesamt mit 200 bezeichnete Hochtemperaturelektrolyse zur Bereitstellung von Wasserstoff, eine insgesamt mit 300 bezeichnete Kohlendioxidbereitstellung und eine insgesamt mit 400 bezeichnete Methanolreinigung.

Die Hochtemperaturelektrolyse 200 wird unter Verwendung einer Elektrolyseeinheit 210 mit einer Festoxidelektrolysezelle mit einer Anodenseite A und einer Kathodenseite C oder einem entsprechenden Zellstapel aus mehreren Festoxidelektrolysezellen bzw. einer Anordnung aus mehreren Zellstapeln durchgeführt. Dazu wird Speisewasser 201 mit Rückführwasser 202 aus einer Wasserstoffverdichtung 220 vereinigt, einer Wasserbehandlung 230 unterworfen und einem Dampfsystem 240 zugeführt. Weiteres Wasser 203 wird in der Methanolsynthese 100 zur Kühlung verwendet, dabei unter Erhalt von Mitteldruckdampf 213 verdampft, unter Erhalt von Niederdruckdampf 204 bzw. Dampf auf einem für die Elektrolyseeinheit geeigneten bzw. gewählten Betriebsdruck (ggf. zuzüglich Druckverlusten) entspannt, und ebenfalls dem Dampfsystem 240 zugeführt. Festoxidelektrolysezellen werden derzeit typischerweise nur geringfügig über Atmosphärendruck betrieben; grundsätzlich könnten in künftigen Entwicklungen aber auch höhere Betriebsdrücke (wie etwa 5 bar) erreicht werden. Dementsprechend könnte ein Druck einer Dampftrommel in dem Dampfsystem 240 dann auf dem Druckniveau liegen entspannt Wenngleich hier nicht konkret veranschaulicht, kann das Wasser zur Kühlung je nach Wasserqualität ebenfalls der Wasserbehandlung 230 unterworfen werden, da es in vergleichbarer Qualität vorliegen muss wie das Prozesswasser. Dampf bei 3.9 bar oder weniger (oder einem beliebigen anderen Druckwert) kann in dem Dampfsystem 240 in eine Dampftrommel eingespeist werden. Mittels des Dampfsystems 240 bereitgestellter Prozessdampf 205 wird mit Rückführwasserstoff 206 aus der Wasserstoffverdichtung 220 beaufschlagt und der Kathodenseite C der Elektrolyseeinheit 210 zugeführt. Die Elektrolyseeinheit 210 wird unter Verwendung einer Spannungsversorgungseinheit 250 betrieben und an der Anodenseite A mit Luft 208 gespült. Ein an der Kathodenseite C der Elektrolyseeinheit 210 entnommenes Elektrolyserohprodukt 207 wird der Wasserstoffverdichtung 200 zugeführt, wobei Wasserstoff 209 bereitgestellt wird. Dieser kann in einen optionalen Wasserstoffspeicher 260 eingespeist werden.

Die Kohlendioxidbereitstellung 300 umfasst eine Kohlendioxidverdichtung 310, der neben Frischkohlendioxid 301 auch Rückführströme 302, 303, die auch weitere Komponenten außer Kohlendioxid enthalten können, zugeführt werden. In der Kohlendioxidverdichtung 310 verdichtetes Kohlendioxid 304 kann in einen optionalen Kohlendioxidspeicher 320 eingespeist werden.

In der Methanolsynthese 100 wird eine Syntheseeinheit 110 verwendet, die insbesondere einen oder mehrere, mittels des Wassers 203 gekühlte Rohrreaktoren aufweisen kann. Die Syntheseeinheit 110 kann unter Erhalt von Mitteldruckdampf 213 gekühlt werden. Der Syntheseeinheit wird ein Speisestrom 101 zugeführt, der unter Verwendung des Wasserstoffs 209 und des Kohlendioxidstroms 304 und eines Rückführstroms 102 gebildet und in einem Feed-Effluent-Wärmetauscher 120 und danach in einem Spitzenerhitzer 130 erwärmt wird. Ein Produktgemisch 103 aus der Syntheseeinheit 110 wird in dem Feed-Effluent-Wärmetauscher 120 und einem Wärmetauscher 140, der auch der Hochtemperaturelektrolyse 200 zugeordnet sein kann, abgekühlt. Eine weitere Abkühlung kann unter Verwendung eines Kühlers 150 erfolgen. Auf diese Weise kann ein Zweiphasenstrom gebildet werden, der in einem Separator 160 in eine Flüssigphase 104 und eine Gasphase 105 getrennt werden kann. Die Flüssigphase 104 wird der Methanoltrennung 400 zugeführt, wobei die Unterteilung der hier dargestellten Verfahrensschritte in Methanolsynthese 100 und Methanoltrennung 400 beliebig ist. Die Gasphase 105 wird zu einem Teil mittels eines Verdichters bzw. Gebläses 170 als Rückführstrom 102 zurückgeführt, ein weiterer Teil wird als Spülgas 106 aus dem Verfahren 1000 ausgeleitet.

Die Methanoltrennung umfasst einen Flashbehälter 410, in den die Flüssigphase 104 aus der Methanolsynthese 100 geflasht wird. Hier gebildetes Flashgas 401 kann in die Kohlendioxidbereitstellung 300 zurückgeführt werden, wie bereits zu dem Rückführstrom 303 oben erläutert. Eine verbleibende Flüssigphase 402 wird in eine Rektfiziereinheit 420 in Form einer oder mehrere, dann parallel betriebener Rektifikationskolonnen, geflasht. Die Rektfiziereinheit 420 wird unter Verwendung eines Sumpfverdampfers 430 und eines Kopfkondensators 440 mit einem Abscheidebehälter 450 in an sich für Rektifikationskolonnen bekannter Art betrieben. Nicht kondensierendes Kopfgas 403 der Rektfiziereinheit 420 wird in Form des erwähnten Rückführstroms 302 zurückgeführt. Am Sumpf der Rektfiziereinheit 420 wird Abwasser 404 ausgeführt. Ein Methanolprodukt 405 und Fuselöle 406 werden aus der Rektfiziereinheit 420 als Seitenströme ausgeführt.

Somit wird gemäß Figur 1 in dem Verfahren 1000 durch eine Hochtemperaturelektrolyse von Wasser Wasserstoff 209 erzeugt, der insbesondere bei vergleichsweise niedrigem Druck vorliegt. Der Wasserstoff 209 wird mit importiertem Kohlendioxid 301 und ggf. einem Kohlendioxidrecycle gemischt und der Methanolsynthese 100 zugeführt. Bei Bedarf kann eine Komprimierung erfolgen. Der Niederdruckdampf 204, der durch die Kühlung der hier isotherm betriebenen Reaktionseinheit 110 erzeugt wird, kann der Elektrolyseeinheit 210 zugeführt werden. Kohlendioxidreiches Abgas 403 bzw. 302 vom Kopf der Rektifikationseinheit 420 kann der Kohlendioxidverdichtung 310 zugeführt werden. Wenn Kohlendioxid 301 bereits mit hohem Druck geliefert wird, kann auch eine Rückführung zum Wasserstoffverdichter 220 erfolgen oder eine kleinere Kompressionseinheit kann separat in diesem Strom installiert werden. Der Wasserstofftank 260 kann nach der Wasserstoffverdichtung 220 installiert werden, um eine gewisse Entkopplung der Hochtemperaturelektrolyse 200 und der Methanolsynthese 100 zu bewirken. Es sind verschiedene Tank- und Kompressorkonfigurationen möglich. Beispielsweise kann ein druckbeaufschlagter Tank neben einem weiteren Tank mit konstantem Volumen bereitgestellt werden, so dass die Wasserstoffverdichtung 220 den Druckabfall ausgleichen muss, was einen Speicherdruck ermöglicht, der unter dem Druck in der Methanolsynthese liegt. In ähnlicher Weise kann ein Kohlendioxidspeicher 320 optional nach der Kohlendioxidverdichtung 310 installiert werden. Auch kann beispielsweise flüssiges Kohlendioxid zum Einsatz kommen.

Im Falle einer Verwendung einer Elektrolyse mit Protonenaustauschmembran kann der Gasstrom 403 aus der Rektifikationseinheit 420 rückgeführt und analog verdichtet werden. Auch ein Recycle 301 bzw. 401 aus einer Flasheinheit 410 kann verwendet werden. Für die Elektrolyse mit Protonenaustauschmembran wird kein Dampf benötigt, stattdessen kann Dampf integriert werden, um den Betrieb des Sumpfverdampfers 430 der Rektifikationseinheit 420 abzudecken, als Exportdampf genutzt werden oder einen Verdichter über eine Turbine antreiben.

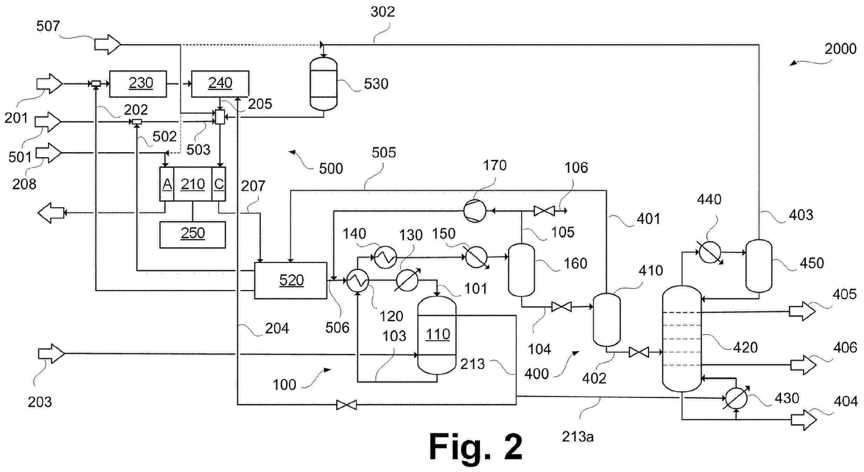

In Figur 2 ist ein Verfahren 2000 gemäß einer weiteren Ausgestaltung der Erfindung veranschaulicht, das im Gegensatz zu dem in Figur 1 veranschaulichten Verfahren 1000 eine Hochtemperaturkoelektrolyse von Wasser und Kohlendioxid umfasst. Diese ist der besseren Unterscheidbarkeit halber mit 500 bezeichnet.

Die Hochtemperaturkoelektrolyse 500 wird unter Verwendung einer Elektrolyseeinheit 210 betrieben. Diese weist eine Festoxidelektrolysezelle mit einer Anodenseite A und einer Kathodenseite C oder einen entsprechenden Zellstapel aus mehreren Festoxidelektrolysezellen bzw. eine Anordnung aus mehreren Zellstapeln auf. Speisewasser 201 wird mit Rückführwasser 202 aus einer Elektrolyseproduktverdichtung 520 vereinigt und einer Wasserbehandlung 230 unterworfen sowie einem Dampfsystem 240 zugeführt. Weiteres Wasser 203 wird in der Methanolsynthese 100 zur Kühlung verwendet, unter Erhalt von Mitteldruckdampf verdampft, auf Niederdruckdampf 204 entspannt und ebenfalls dem Dampfsystem 240 zugeführt. Kohlendioxid 501 wird mit einem Rückführstrom 502 aus der Elektrolyseproduktverdichtung 520 zum Aufrechterhalten reduzierender Bedingungen unter Erhalt eines Sammelstroms 503 vereinigt. Mittels des Dampfsystems 240 bereitgestellter Prozessdampf 205 wird mit dem Sammelstrom 503 und ggf. weiteren Stoffströmen vereinigt der Kathodenseite C der Elektrolyseeinheit 210 zugeführt. Die Elektrolyseeinheit 210 wird unter Verwendung einer Spannungsversorgungseinheit 250 betrieben und an der Anodenseite A mit Luft 208 gespült. Ein an der Kathodenseite C der Elektrolyseeinheit 210 entnommenes Elektrolyserohprodukt 507 wird der Elektrolyseproduktverdichtung 520 zugeführt, wobei das Rückführwasser 202 und der Rückführstrom 502 gebildet werden. Der Elektrolyseproduktverdichtung 520, die insbesondere mehrstufig durchgeführt wird, wird auch ein Rückführstrom 505 zugeführt, der insbesondere dem Stoffstrom 401 des Verfahrens 1000 gemäß Figur 1 entsprechen kann. Es wird ferner ein Wasserstoff, Kohlenmonoxid und Kohlendioxid enthaltendes (und ggf. Nebenkomponenten aufweisendes) Synthesegas 506 bereitgestellt.

Die Methanolsynthese 100 und Methanoltrennung 400 können in dem Verfahren 2000 gemäß Figur 2 im Wesentlichen, wie zuvor zu Verfahren 1000 gemäß Figur 1 erläutert, durchgeführt werden. Auf die obigen Ausführungen wird verwiesen.

Wie in Figur 2 veranschaulicht, kann in dem Verfahren 2000 ein extern bereitgestelltes Gasgemisch 507, bspw. ein Kohlendioxid enthaltendes Abgas, verwendet und, je nach Zusammensetzung, dem Prozessdampf 205, und damit kathodenseitig der Elektrolyseeinheit 210, anodenseitig der Elektrolyseeinheit 210 oder zunächst in eine Präreformiereinheit 530 eingespeist werden. Auch letztere ist optional vorhanden und ihr kann ein den Stoffströmen 302 bzw. 403 entsprechender Stoffstrom zugeführt werden. Ein der Präreformiereinheit 530 entnommener Gasstrom kann auch zumindest zu einem Teil der Anodenseite A zugeführt werden.

In der Ausgestaltung des Verfahrens 2000 gemäß Figur 2 wird das kohlendioxidreiche Abgas 403 aus der Rektifikationseinheit 420 in die Hochtemperaturkoelektrolyse 500 zurückgeführt, die unter Beimischung des Recyclestroms 505 das Synthesegas 506 erzeugt. Das Synthesegas 506 wird dann in der Methanolsynthese 100 in Methanol umgewandelt. Mitteldruckdampf 213, der wie zuvor durch die Kühlung in der Methanolsynthese 100 erzeugt wird, kann der Hochtemperaturkoelektrolyse 500 nach Entspannung als Niederdruckdampf 204 zugeführt werden. Da die Reaktion von Synthesegas zu Methanol (siehe Gleichung (1) oben) aber exothermer ist als jene von Kohlendioxid (siehe Gleichung (2) oben), wird mehr Mitteldruckdampf 213 erzeugt als in dem Verfahren 1000 gemäß Figur 1. Der überschüssige Mitteldruckdampf 213 kann, wie mit 213a veranschaulicht, zur Beheizung des Sumpfverdampfers 430 der Rektifikationseinheit 420 verwendet werden. Kohlendioxidreiches Abgas 403 aus dem Kolonnenkopf der Rektifikationseinheit 420 kann zur Hochtemperaturkoelektrolyse rezykliert werden und auf diese Weise im System verbleiben. Die Präreformierung des Abgases in der Präreformiereinheit 530 kann vorteilhaft sein, um das Risiko einer Zelldegradation zu vermeiden. Optional kann, wie erwähnt, Erdgas 507 oder externes Abgas importiert und der Kathodenseite C der Elektrolyseeinheit 210 zugeführt werden. Auch für diesen Importstrom könnte eine Vorreformierung vorteilhaft sein. Eine teilweise Einspeisung dieses Stroms in die Anodenseite A zur Erzeugung von Wärme und zur Senkung des elektrischen Energiebedarfs kann für beide Verfahren 1000 und 2000 gemäß den Figuren 1 und 2 vorteilhaft sein.

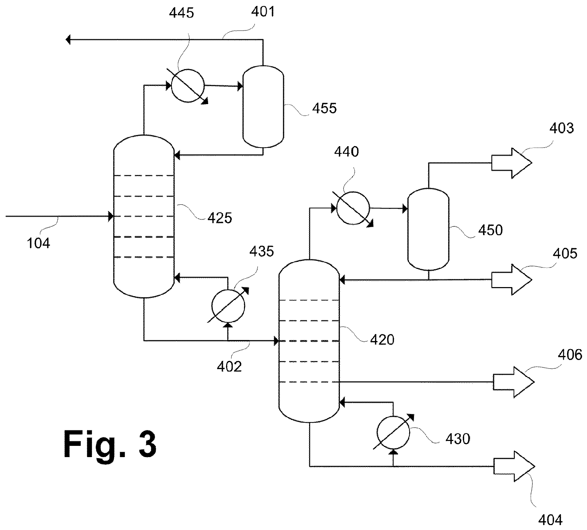

Kann mit dem Synthesegaseinsatz aus der Hochtemperaturkoelektrolyse 500 wegen höherer Nebenproduktbildung kein Methanol ausreichender Qualität erzielt werden, oder in anderen Fällen, d.h. in Ausgestaltungen gemäß Figur 1 und Figur 2, kann ein Zweikolonnenaufbau gemäß Figur 3 eingesetzt werden. Wie hier veranschaulicht, ist zusätzlich zur Rektifikationseinheit 420, die damit als Raffinationskolonne dient, eine weitere Rektifikationseinheit 425 mit Sumpfverdampfer 435, Kopfkondensator 445 und Abscheidebehälter 455 vorgeschaltet, wobei die Einbindung mit den wie zuvor bezeichneten Stoffströmen veranschaulicht ist. Die Rektifikationseinheit 425 kann mit weiteren Stoffströmen gespeist werden. In den zuvor und hier veranschaulichten Ausgestaltungen kann beispielsweise stromab des Abscheiders 160 ein weiterer Flash im Strom 105 folgen, dessen Flüssigphase auch in die Rektifikationseinheit 425, ggf. auf einer anderen Stufe, eingespeist wird.

Wenngleich hier nicht gesondert veranschaulicht, kann vor dem Eintritt in dir Rektifikationseinheit 425 je nach Flashanordnung auch ein Ventil angeordnet sein, da der Strom 104 bzw. ein anderer in die Rektifikationseinheit 425 eingespeister Stoffstrom noch auf hohem Druck vorliegen kann.

## Patentansprüche

1. Verfahren (1000, 2000) zur Herstellung von Methanol, bei dem ein Wasserstoff und Kohlendioxid enthaltender Methanolsyntheseeinsatz (101) unter Bildung eines Methanol, tiefer als Methanol siedende Komponenten und höher als Methanol siedende Komponenten enthaltenden Rohproduktgemischs (103) einer Methanolsynthese (100) unterworfen wird, wobei zumindest ein Teil des Rohproduktgemischs (103) unter Bildung eines Methanolprodukts (405) und einer oder mehrerer, gegenüber dem Rohproduktgemisch (103) an Methanol abgereicherter und an zumindest einer der tiefer als Methanol siedenden Komponenten angereicherter Leichtgasfraktionen (401, 403) einer Methanolaufreinigung (400) unterworfen wird, wobei zumindest ein Teil des Wasserstoffs in dem Methanolsyntheseeinsatz (101) unter Verwendung einer Elektrolyse (200, 500) bereitgestellt wird, und wobei zumindest ein Teil der Leichtgasfraktion (401, 403) oder zumindest ein Teil zumindest einer der mehreren Leichtgasfraktionen (401, 403) in die Elektrolyse (200, 500) oder einen weiteren, zur Bereitstellung des Methanolsyntheseeinsatzes (101) verwendeten Verfahrensschritt (310) zurückgeführt wird.

2. Verfahren (1000, 2000) nach Anspruch 1, bei dem die Elektrolyse (200, 500) als Hochtemperaturelektrolyse durchgeführt wird, der Dampf (205) zugeführt wird.

3. Verfahren (1000, 2000) nach Anspruch 2, bei dem der Dampf (205) einen Dampfanteil umfasst, der unter Verwendung von Dampf (204) gebildet wird, der bei einer Kühlung einer in der Methanolsynthese (100) eingesetzten Methanolsyntheseeinheit (110) aus Kühlwasser (203) erzeugt wird.

4. Verfahren (1000) nach einem der vorstehenden Ansprüche 2 oder 3, bei dem die Elektrolyse (200) als Hochtemperaturelektrolyse von Wasser durchgeführt wird, wobei der Methanolsyntheseeinsatz (101) unter Verwendung der Elektrolyse (200) erzeugten Wasserstoff (209) und nicht der Elektrolyse (200) unterworfenes, verfahrensextern bereitgestelltes Kohlendioxid (301) umfasst.

5. Verfahren (1000) nach Anspruch 4, bei dem das verfahrensextern bereitgestellte Kohlendioxid (301) einer Kohlendioxidverdichtung (310) unterworfen wird, wobei zumindest ein Teil der Leichtgasfraktion (401, 403) oder zumindest ein Teil zumindest einer der mehreren Leichtgasfraktionen (401, 403) in die Kohlendioxidverdichtung (301) zurückgeführt wird.

6. Verfahren (2000) nach einem der vorstehenden Ansprüche 2 oder 3, bei dem die Elektrolyse (500) als Hochtemperaturkoelektrolyse von Wasser und Kohlendioxid durchgeführt wird, wobei unter Verwendung der Elektrolyse (500) ein Wasserstoff, Kohlenmonoxid und Kohlendioxid enthaltendes Synthesegas (506) bereitgestellt und zur Bereitstellung des Methanolsyntheseeinsatzes (101) verwendet wird.

7. Verfahren (2000) nach Anspruch 6, bei dem zumindest ein Teil der Leichtgasfraktion (401, 403) oder zumindest ein Teil zumindest einer der mehreren Leichtgasfraktionen (401, 403) der Hochtemperaturkoelektrolyse von Wasser und Kohlendioxid kathodenseitig und/oder anodenseitig, insbesondere nach einer Präreformierung, zugeführt wird.

8. Verfahren (2000) nach einem der vorstehenden Ansprüche 6 oder 7, bei dem zumindest ein Teil der Leichtgasfraktion (401, 403) oder zumindest ein Teil zumindest einer der mehreren Leichtgasfraktionen (401, 403) vor der kathodenseitigen Zuführung zu der Hochtemperaturkoelektrolyse einem Präreformierungsschritt (530) unterworfen wird.

9. Verfahren (2000) nach einem der vorstehenden Ansprüche 6 bis 8, bei dem Erdgas (507) oder ein anderes kohlenwasserstoffhaltiges Gasgemisch zugeführt wird, das kathoden- und/oder anodenseitig in die Hochtemperaturkoelektrolyse eingespeist wird und optional zunächst einem Präreformierungsschritt (530) unterworfen wird.

10. Verfahren (1000, 2000) nach einem der vorstehenden Ansprüche, bei dem die Methanolaufreinigung (400) unter Verwendung einer einzigen oder mehrerer, parallel betriebener Rektifikationseinheiten (420) durchgeführt wird, der oder denen das Methanolprodukt (405) über einen Seitenabzug entnommen wird.

11. Verfahren (1000, 2000) nach Anspruch 10, bei dem der einen oder den mehreren Rektifikationseinheiten (420) eine Flasheinheit (410) vorgeschaltet ist, in die ein bei einer Abkühlung des Rohproduktgemischs (103) gebildetes Kondensat (104) unter Erhalt eines Flashgases (401) und einer Flüssigfraktion (402) geflasht wird, wobei zumindest ein Teil der Flüssigfraktion (402) der einen oder den mehreren Rektifikationseinheiten (420) zugeführt wird.

12. Verfahren (1000, 2000) nach Anspruch 10 oder 11, bei dem ein Kopfgas der einen oder der mehreren Rektifikationseinheiten (420) unter Verbleib einer Gasphase teilkondensiert wird, wobei zumindest ein Teil der Gasphase die oder eine der Leichtgasfraktionen (401, 403) ist.

13. Verfahren (1000, 2000) nach einem der vorstehenden Ansprüche 10 bis 12, bei dem der einen oder den mehreren Rektifikationseinheiten (420) unterhalb des Methanolprodukts über einen weiteren Seitenabzug ein Fuselöle enthaltender Strom (406) und sumpfseitig Abwasser (404) entnommen werden.

14. Anlage zur Herstellung von Methanol, die dafür eingerichtet ist, einen Wasserstoff und Kohlendioxid enthaltenden Methanolsyntheseeinsatz (101) unter Bildung eines Methanol, tiefer als Methanol siedende Komponenten und höher als Methanol siedende Komponenten enthaltenden Rohproduktgemischs (103) einer Methanolsynthese (100) zu unterwerfen, zumindest einen Teil des Rohproduktgemischs (103) unter Bildung eines Methanolprodukts (405) und einer oder mehrerer, gegenüber dem Rohproduktgemisch (103) an Methanol abgereicherter und an zumindest einer der tiefer als Methanol siedenden Komponenten angereicherter Leichtgasfraktionen (401, 403) einer Methanolaufreinigung (400) zu unterwerfen, zumindest einen Teil des Wasserstoffs in dem Methanolsyntheseeinsatz (101) unter Verwendung einer Elektrolyse (200, 500) bereitzustellen, und zumindest einen Teil der Leichtgasfraktion (401, 403) oder zumindest ein Teil zumindest einer der mehreren Leichtgasfraktionen (401, 403) in die Elektrolyse (200, 500) oder einen weiteren, zur Bereitstellung des Methanolsyntheseeinsatzes (101) verwendeten Verfahrensschritt (310) zurückzuführen.

15. Anlage nach Anspruch 14, die zur Durchführung eines Verfahrens nach einem der Ansprüche 1 bis 13 eingerichtet ist.
